# EUROPEAN PATENT SPECIFICATION

(11) **EP 1 390 529 B1**
(45) Date of publication and mention of the grant of the patent: **29.09.2004**
(21) Application number: 02704676.2
(22) Date of filing: 29.01.2002
(51) Int. Cl.: C12Q 1/68

(54) **METHOD OF ANALYSING DNA METHYLATION USING FLUORESCENCE POLARISATION**
VERFAHREN ZUR ANALYSE DER DNA-METHYLIERUNG UNTER VERWENDUNG VON FLUORESZENZPOLARISATION
PROCEDE D'ANALYSE DE LA METHYLATION D'ADN PAR POLARISATION DE FLUORESENCE

(30) Priority: 29.01.2001 DE 10104937
(43) Date of publication of application: 25.02.2004
(73) Proprietor: Epigenomics AG, 10435 Berlin (DE)
(72) Inventor: BERLIN, Kurt, 14532 Stahnsdorf (DE)
(74) Representative: Schubert, Klemens, Dr.
(86) International application number: PCT/EP2002/000922
(87) International publication number: WO 2002/061123

(56) References cited:
- EP-A- 0 382 433
- WO-A-00/11220
- WO-A-00/70090
- WO-A-99/28498

## Description

### Field of the invention

This invention relates to a method for the analysis of methylation patterns in genomic DNA, for use in high throughput analysis, research or clinical settings. This method utilises bisulfite treatment and fluorescence polarisation assay techniques.

### Background of the invention

The levels of observation that have been studied in recent years in molecular biology have concentrated on genes, the translation of those genes into RNA, and the transcription of the RNA into protein. There has been a more limited analysis of the regulatory mechanisms associated with gene control. Gene regulation, for example, at what stage of development of the individual a gene is activated or inhibited, and the tissue specific nature of this regulation is less understood. However, it can be correlated with a high degree of probability to the extent and nature of methylation of the gene or genome. From this observation it is reasonable to infer that pathogenic genetic disorders may be detected from irregular genetic methylation patterns.

### State of the art

### Methylation and disease

The efforts of the Human Genome project are concentrated on the sequencing of the human genome. It is expected that this will yield considerable therapeutic and diagnostic benefits for the treatment of disease. However, these efforts have so far been unable to address a significant aspect of genetic disorders, the epigenetic factor. The epigenetic regulation of gene transcription has been shown to effect many disorders. One of the most significant epigenetic mechanisms so far identified has been the methylation of cytosine. The methylation of cytosine at the 5 position is the only known modification of genomic DNA. Although the exact mechanisms by which DNA methylation effects DNA transcription are unknown, the relationship between disease and methylation has been well documented. In particular, methylation patterns of CpG islands within regulatory regions of genome appear to be highly tissue specific. Therefore, it follows that mis-regulation of genes may be predicted by comparing their methylation pattern with phenotypically 'normal' expression patterns. The following are cases of disease associated with modified methylation patterns.
- Head and neck cancer (Sanchez-Cespedes M et al. "Gene promoter hypermethylation in tumours and serum of head and neck cancer patients" Cancer Res. 2000 Feb. 15;60 (4):892-5)
- Hodgkin's disease (Garcia JF et al "Loss of p16 protein expression associated with methylation of the p16INK4A gene is a frequent finding in Hodgkin's disease" Lab invest 1999 Dec; 79 (12):1453-9)
- Gastric cancer (Yanagisawa Y et al. "Methylation of the hMLH1 promoter in familial gastric cancer with microsatellite instability" Int J Cancer 2000 Jan 1; 85 (1):50-3)
- Prader-Willi/Angelman's syndrome (Zeschnigh et al "Imprinted segments in the human genome: different DNA methylation patterns in the Prader Willi/Angelman syndrome region as determined by the genomic sequencing method" Human Mol. Genetics (1997) (6) 3 pp 387-395)
- ICF syndrome (Tuck-Muller et al "CMDNA hypomethylation and unusual chromosome instability in cell lines from ICF syndrome patients" Cytogenet Call Genet 2000; 89(1-2):121-8)
- Dermatofibroma (Chen TC et al "Dermatofibroma is a clonal proliferative disease" J Cutan Pathol 2000 Jan;27 (1):36-9)
- Hypertension (Lee SD et al. " Monoclonal endothelial cell proliferation is present in primary but not secondary pulmonary hypertension" J clin Invest 1998 Mar 1, 101 (5):927-34)
- Autism (Klauck SM et al. "Molecular genetic analysis of the FMR-1 gene in a large collection of autistic patients" Human Genet 1997 Aug; 100 (2) : 224-9)
- Fragile X Syndrome ( Hornstra IK et al. " High resolution methylation analysis of the FMR1 gene trinucleotide repeat region in fragile X syndrome" Hum Mol Genet 1993 Oct, 2(10):1659-65)
- Huntigton's disease (Ferluga J et al. "possible organ and age related epigenetic factors in Huntington's disease and colorectal carcinoma" Med hypotheses 1989 May; 29 (1) ; 51-4)

The state of the art covers two basic methods for the analysis of methylation patterns and nucleic acids. The first concerns a method for the analysis of methylation patterns at specific sites in the genome. The second concerns a method that utilises fluorescent polarisation for the analysis of nucleic acids.

### Detection of cytosine methylation in DNA.

The modification of the genomic base cytosine to 5-methylcytosine represents the epigenetic parameter which to date is the most examined and understood. Nevertheless, the characterisation of this epigenomic parameter is still not on par with that of genotyping of cells and individuals. There is still room for the development of more methodologies for the high throughput analysis and characterisation of the methylation patterns of cells. The most comprehensive patent covering this field, is WO 99/28498 . Said invention providing a means for the detailed analysis of methylation patterns. The disclosed invention aims to provide an alternative solution by utilising fluorescence polarisation techniques in the analysis of methylation patterns. It will provide a simple methylation assay especially suitable for a medium throughput clinical environment.

Standard methods of sequence analysis such as cloning and PCR are insufficient for the analysis of methylation as covalent modifications to the DNA such as methylation are not conserved.

There are currently three methods used for the differentiation of 5-methyl cytosine from unmethylated cytosine in DNA sequence.

The first method uses restriction enzymes. Restriction endonucleases cut DNA sequences at specific locations, upon recognition of a specific sequence, usually 4-8 bases in length. These enzymes are highly specific as to the sequence they recognise. In some cases, known as 'methylation sensitive' they will not cut at the methylated version of the recognition sequence. Therefore methylation sensitive enzymes can be used to identify methylation within restriction enzyme sites.

The position of the cuts may be determined by gel electrophoresis, followed by blotting and hybridisation. This method has not proved useful for the efficient identification of methylated CpG sites in the genome for two reasons. Firstly, most CpG islands that are methylated are not within the recognition sequence of most restriction enzymes. Secondly, the sensitivity of this method is extremely low (Bird, A.P., Southern, E.M., J.Mol.Biol. 118,27-47). The sensitivity can be improved by amplifying the region after restriction exonuclease digestion. Two primers are used that flank the recognition site of the enzyme. In the event of the digestion taking place amplification will not occur. The amplification products can then be analysed by blotting and hybridisation to identify the site of the cut. In theory the resolution of this technique can be one base pair. However, as it is highly labour intensive and costly it is not a practical solution to the large scale analysis of methylation patterns. (Shemer, R. Et al., PNAS 93, 6371- 6376)

The second method utilises the sequencing method developed by Maxam Gilbert, for 5-methyl cytosine identification. The technique involves the partial chemical cleavage of whole DNA followed by ligation, amplification and hybridisation. In theory regions having a size of less than 1000 base pairs can be analysed. However, this method is so complicated and unreliable that it is rarely used.

A third method utilises bisulfite treatment of genomic DNA followed by Taqman analysis of the modified DNA (WO 00/700 90)

### Bisulfite treatment

The preferred method of methylation analysis involves a chemical modification of the DNA sequence. The method is based on the bisulfite conversion of cytosine to uracil. DNA is denatured and then treated using a bisulfite solution. This results in the conversion of cytosine to uracil, that leaves the methylated cytosines unmodified. Uracil acts as analogue of thymine for base pairing purposes, rather than cytosine. As a result of the bisulfite treatment the DNA strands that were originally complementary to each other, the coding and template strands are no longer complimentary.

Oligonucleotide primers for the amplification of each bisulfite treated strand can then be designed. Enzymatic amplification of the sequence results in the incorporation of thymine nucleotides at positions that were cytosine in the original sequence.

Amplification of the bisulfite treated DNA using bisulfite specific primers results in the formation of a complementary strand, the sequence of which is dependant on the methylation status of the genomic sample, and is thus unique from the original pre bisulfite treated complementary strand. The bisulfite treatment and subsequent amplification therefore results in the formation of 4 unique nucleic acid fragments. These four strands all contain the same information, assuming that methylation has been symmetric, that is, both strands of the CpG position have been methylated. The methylation status of each CpG position may therefore be assessed independently four times.

Current methods for the assessment of the methylation status of a CpG position bisulfite converted sequence include standard chromatographic analysis, hybridisation analysis, or mass spectrometry analysis.

All methods require the purification of the PCR products, for example by gel electrophoresis which may also serve directly for analysis. In the hybridisation analysis the bisulfite treated and PCR amplified nucleic acids are chemically labelled and hybridised to complementary oligonucleotides. The amplified fragments are tested using two labelled oligonucleotides, one which is specific for unmethylated DNA, and therefore is CpG rich, and another specific for methylated DNA which contains no CpG. The hybridisation is then detected by an assay for the label. This form of analysis may be carried out in the form of a DNA array, allowing high throughput analysis. An alternative method, utilising MALDI mass spectrometer analysis of nucleic acids has been described by Kirpekar F et. al. 'DNA sequence analysis by MALDI mass spectrometry ' Nucleic Acid Research;26, 2354-9.

### Fluorescence assays

The disclosed method provides a new use for an established form of fluorescence assay to provide a novel solution to the problem of analysis of chemically modified methylated genomic DNA sequence.

The use of fluorescence techniques for the analysis of small biomolecules is well known. There are currently four commercially available methods for the closed tube luminescence analysis of enzymatic amplification products. These are the Taqman, Molecular Beacons, LightCycler and Amplifluor assays. All are based on the use of fluorescence resonance energy transfer (FRET). FRET is a form of molecular energy transfer whereby energy is passed between donor and acceptor species. Energy is passed non radiativley between an acceptor molecule and a donor molecule. The donor absorbs a photon and passes this non radiatively to the acceptor molecule, thus causing it to fluoresce. When two fluorophores whose excitation and emission spectra overlap are in close proximity, excitation of one fluorophore will cause it to emit light at wavelengths that are absorbed by and that stimulate the second fluorophore, causing it in turn to fluoresce.

All methods based on FRET are characterised by relatively high signal-to-noise ratios and a good ability to discriminate between positive and negative reactions. However, they are all limited in the sense that either a dual Label probe or primer or two separate probes per target have to be used. This seriously complicates probe design and synthesis. In addition, since they all employ labels with rapidly decaying fluorescence and broad emission peaks, the possibilities for multiplex detection are limited. The invention proposes the use of fluorescence polarisation as opposed to FRET.

### Fluorescence Polarisation

Most fluorescence assays utilise the fluorescence transfer properties of donor and acceptor groups to observe the properties of small biomolecules. The use of fluorescence polarisation techniques was, until recently, limited to smaller analytes in the region of a molecular weight of about 1,000 Daltons. It had been utilised mainly in a number of immunoassays and for the measurement of microviscosity and molecular volume. One of the main advantages of fluorescence polarisation techniques over other methods is that it allows the analysis of homogenous solutions, i.e. there is no need for purification procedures.

The concept of fluorescence polarisation has been known since the 1920s. It is a measure of the time-average rotational motion of fluorescent molecules.

The fluorescence polarisation technique allows the observation of changes in the rotational properties of molecules in a solution. Molecules in solution rotate and tumble about multiple axis. Fluorescence polarisation relies on the property of plane polarised light to be emitted by a stationary fluorescent molecule. If plane polarised light is used to irradiate a fluorescent molecule, the molecule will emit plane polarised light between excitation and emission only when stationary. Larger molecules, i.e. those of larger molecular weight and/or volume tumble more slowly about their axes than smaller molecules. As the degree of polarisation of the light emitted by the fluorescent molecule is related to the degree of movement of the molecule, it is possible to distinguish between larger and smaller molecules based on the degree of polarisation of light.

In fluorescence polarisation techniques, the fluorescent molecule is first excited by polarised light. The polarisation of the emission is measured by measuring the relative intensities of emission (i) parallel to the plane of polarised excitation light and (ii) perpendicular to the plane of polarised excitation light. A change in the rate of tumbling due to a change in size and/or rigidity is accompanied by a change in the relationship between the plane of excitation light and the plane of emitted fluorescence, i.e., a change in fluorescence polarisation. The observed FP of a species is described by the Perrin equation and is related to the ratio of the rotational relaxation time and the fluorescent lifetime.

Fluorescence polarisation (hereafter referred to as FP) is expressed as a ratio of polarised to non polarised light. As such, it has a distinct advantage over other forms of fluorescence detection in that it is independent of the initial concentration of fluorescence in the solution. As long as the amount of fluorescence is still significantly detectable accurate results can be given. The FP difference between totally bound and totally unbound DNA represents the complete dynamic range of FP. As long as a statistically significant difference can be derived from the interaction of low molecular fluorophore labelled nucleic acids and those hybridised to larger nucleic acid molecules FP can be a suitable method for the detection of chemical interactions. However, due to the effects of the local motion of fluorophores it may not always possible to predict the values for suitable probes, and they may have to be empirically derived.

For a system in which a fluorophore is attached to a nucleic acid of low molecular weight or volume, and is then hybridised to a larger nucleic acid the observed fluorescence (P) may be described as follows:

**P = P**_{**max**} **[OLIGO]b + P**_{**min**} **([OLIGO]i - [OLIGO]b)**

where Pₘₐₓ is the polarisation observed for fluorescence labelled oligonucleotides that have been hybridised to the larger nucleic acid. Pₘᵢₙ is the polarisation observed from the unincorporated dye labelled oligonucleotides,
where [OLIGO]i is the initial concentration of fluorescent dye labelled oligonucleotides and [OLIGO]b is the concentration of incorporated dye labelled oligonucleotides.

It is to be understood that fluorescence polarisation includes all methods of analysis of polarised light emitted from a fluorophore group attached to a dNTP or combined in polynucleotide group. This is state of the art and is described by M.E.Jolley, J.Analytical Toxicology 1981 (5) 236-240 which is hereby incorporated for reference.

The application of FP techniques to nucleic acid analysis was disclosed in patent application EP0382433B1, which is hereby incorporated for reference. The use of FP for nucleic acid sequence analysis has been disclosed in patent publications WO 92/18650 and WO 00/11220, which are hereby incorporated for reference and is known in the state of the art. However, the use of fluorescence polarisation as a tool for the analysis of DNA methylation patterns is unknown. The problem of the invention lies in the analysis of this specialised form of nucleic acid sequence. The methods of analysis currently only possible using chromatography, hybridisation and MALDI mass spectrometer techniques.

### Summary of the invention

The invention is a method for the detection of DNA methylation patterns. The state of the art consists of several methods for the analysis of bisulfite converted genomic sequence. However, all entail a two step procedure whereby the bisulfite conversion is followed by a PCR amplification and a subsequent analysis. All current methods of analysis require the purification of nucleic acid products after enzymatic amplification, usually by some form of gel electrophoresis. The present invention provides a significant improvement of the state of the art in that bisulfite sequence analysis may be carried out in a homogenous solution. This allows analysis of the sequence in a closed tube, i.e. concurrent with or upon completion of the enzymatic amplification without need for further purification. In addition the method of the invention may be adapted to other diagnostic formats, for example, high density DNA chip analysis. The method of the invention provides a cost effective method of analysis. Results are obtainable minutes after carrying out the methylation specific reaction.

The proposed invention provides an innovative solution to the problem by providing a novel method comprising the following steps:
a) treatment of nucleic acid sample with a chemical solution in order to convert unmethylated cytosine to Uracil;
b) amplifying said treated nucleic acid using oligonucleotide primers specific for the converted sequence;
c) contacting the amplified sequence with fluorophore labelled oligonucleotide probes;
e) hybridising a fluorophore labelled oligonucleotide probe to the amplified sequence;
f) detecting the fluorescence polarisation of the labelled oligonucleotide probes.

According to a first object of the invention a method for the analysis of the methylation of specific cytosine bases in genomic DNA samples is provided, wherein the following steps are performed:
(a) the genomic DNA is chemically treated in such a manner that cytosine is converted into uracil or a similar acting base regarding the base pairing behaviour in the DNA duplex, 5 methylcytosine however remains basically unmodified;
(b) the chemically treated DNA is amplified using at least one oligonucleotide (type A) as primer in a polymerase reaction, whereby the two strands of the polymerase reaction product are manufactured in unequal quantities;
(c) the amplificate is hybridised with one or more pairs of oligonucleotides (type B), which hybridise to the positions which are to be examined regarding their methylation status in the genomic DNA sample whereby one oligonucleotide of each pair hybridises preferentially in each case if in the genomic DNA sample the position was methylated, while the other oligonucleotide of the pair hybridises preferentially, if the position was unmethylated. Each oligonucleotide of a pair is labelled with a unique fluorescent label;
(d) the fluorescence polarisation characteristics of the solution are measured, whereby for each fluorescent label used one determines the degree of polarisation.

According to the invention it is preferred that the hybridised oligonucleotide is extended by means of deoxynucleotides and a polymerase.

It is alos preferred according to the invention that the fluorescence polarisation of the fluorophore labelled oligonucleotides is measured before hybridisation with the amplificate and again after hybridisation with the amplificate. Therein it is especially preferred that the oligonucleotide hybridisation is detected by an increase in fluorescence polarisation.

It is also preferred that said fluorophore is selected from the group consisting of 5'carboxyfluorescein, 6-carboxy-X-rhodamine, N,N,N',N',-tetramethyl-6-carboxy-X-rhodamine, BODIPY,Texas Red, Cy3, Cy5, FAM, FITC, DAPI, HEX, and TET.

According to the invention it is highly preferred that the DNA sample is cleaved prior to bisulfite treatment with restriction endonucleases.

Accoring to the invention it is also highly preferred and a main feature that the enzymatic amplification of the chemically treated DNA may be such that only one strand of the DNA sample is amplified.

It is also preferred that the DNA sample is cleaved prior to bisulfite treatment with restriction endonucleases.

Preferred is also according to the invention that the DNA sample is isolated from mammalian sources e.g. cell lines, blood, sputum, faeces, urine, cerebrospinal fluid, tissue embedded in paraffin, for example, ocular tissue, intestine, kidney, brain, heart, prostate, lung, chest or liver, histological slides and all possible combinations.

It is also especially preferred that the fluorescence polarisation of the enzymatically amplified DNA is measured directly from the container in which the amplification reaction was carried out.

It is further preferred that the Type B oligonucleotide is immobilised on a surface prior to hybridisation with the amplificate.

It is still further preferred according to the invention that the bisulfite treated DNA is bound to a surface prior to hybridisation with the Type B oligonucleotide. Therein it is also preferred that the surface comprises silicon, glass, polystyrene, aluminium, steel, iron, copper, nickel, silver or gold.

It is also preferred according to the invention that the information generated about the methylation status at the target site is provided to a computing device comprising one or more databases. Preferred is also that the information generated about the methylation status at the target site is provided to a computing device comprising one or more learning algorithms.

Also disclosed is a diagnostic kit for the detection of the methylation of specific cytosine bases in genomic DNA samples comprising one or more pairs of fluorescent labelled oligonucleotides designed to hybridise to at a target site on a DNA template.

### Detailed Description

The methodology according to the invention consists of the following steps:

Firstly the genomic DNA sample must be isolated from tissue or cellular sources. For mammals, more preferably humans, the DNA sample may be taken from any tissue suspected of expressing the target site within the genome. For mammals, more preferably humans, such sources may include cell lines, blood, sputum, faeces, urine, spinal fluid, tissue embedded in paraffin, for example tissue of eyes, intestine, kidney, brain, heart, prostate, lung, chest or liver, histological slides. Extraction may be by means that are standard to one skilled in the art, these include the use of detergent lysates, sonification and vortexing with glass beads. However, in a preferred embodiment the extraction will take place in a minute volume of oil, in order to minimise DNA loss. Once the nucleic acids have been extracted the genomic double stranded DNA is used for analysis.

In a preferred embodiment the DNA may be cleaved prior to the chemical treatment, this may be any means standard in the state of the art in particular with restriction endonucleases.

In a further preferred embodiment the resulting cut ends of the cleaved DNA may be ligated to short double stranded nucleic acid sequences. Said sequences, hereafter known as 'adaptors', may present single stranded projections. The adaptors may be attached, for example, by means of a thermolabile ligase enzyme, such as T4 DNA ligase. The ligase is then heat denatured prior to chemical modification of the DNA sample. The adaptors may be of such sequence that they remain unmodified by the chemical treatment used to distinguish methylated from unmethylated DNA sequence. Said adaptors may be used for the enzymatic amplification of the DNA sample by providing a target for the hybridisation of oligonucleotide primers.

The use of adaptor molecules is well known within the prior art and will not be elaborated upon.

The sample DNA is then treated chemically in order to convert the methylated cytosine bases into uracil. The chemical modification may be by means standard in the state of the art, for example, (but not limited to) treatment with bisulfite solution.

In both cases the treatment results in the conversion of unmethylated cytosine bases to uracil wherein methylated cytosine bases remain unmodified.

Wherein the chemical modification takes the form of a bisulfite treatment of the DNA the following steps may be followed.

The double stranded DNA must be denatured. This may take the form of a heat or chemical denaturation. The heat denaturation may be carried out at variable temperatures. For high molecular weight DNA, the denaturation temperature is generally greater than 90 oC. However, the analysis may be upon smaller fragments which do not require such high denaturing temperatures. In addition, as the reaction proceeds and the cytosine residues are converted to uracil the complementarity between the strands decreases. Therefore, a cyclic reaction protocol may consist of variable denaturation temperatures.

The bisulfite conversion then consists of two important steps, the sulfonation of the cytosine and the subsequent deamination. The equilibra of the reaction are on the correct side at two different temperatures for each stage of the reaction. Taking into account the kinetics of the reactions it is preferable that the reaction takes place under cyclic conditions, with changing temperatures. The temperatures and length at which each stage is carried out may be varied according to the specific requirement of the situation. However, a preferred variant of the method comprises a change of temperature from 4 C (10 minutes) to 50 C (20 minutes). This form of bisulfite treatment is state of the art with reference to WO 99/28498.

Said chemical conversion may take place in any format standard in the the art. This includes but is not limited to modification within agarose gel, in denaturing solvents or within capillaries.

Bisulfite conversion within agarose gel is state of the art and has been described by Olek et al, Nucl. Acids. Res. 1996, 24, 5064-5066. The DNA fragment is embedded in agarose gel and the conversion of cytosine to uracil takes place with hydrogensulfite and a radical scavenger. The DNA may then be amplified without need for further purification steps.

In a further preferred embodiment the DNA conversion may take place without an agarose matrix. The DNA may incubated at increased temperatures with hydrogensulfite and a radical scavenger. Said reaction takes place within an organic denaturing solvent. Examples of denaturing solvents include, but are not limited to, Polyethylene glycol dialkyl polyethylenglycoldialkylether, dioxane and substituted derivatives, urea or derivatives, acetonitrile, primary alcohols, secondary alcohols, tertiary alcohols, DMSO or THF.

In a further embodiment, prior to chemical treatment the DNA sample is transferred into a heatable capillary that is permeable to small molecules. The reaction steps of the chemical modification may then be carried out in the capillary tubes by means of the addition and removal of reagents through connected capillaries.

Subsequent to the chemical treatment the two strands of the DNA may no longer be complementary.

Fractions of the so treated genomic DNA are then enzymatically amplified using oligonucleotide primers. These oligonucleotides which, for example, may be complementary to the adaptor molecules, are hereafter distinguished as type A primers. The length and design of said primers may be specific to the area of the genome to be analysed. As such a wide range of primers are suitable for use in this technique. Such primer design is within the state of the art. The amplification may be such that one strand of the double strands is preferentially amplified, i.e. that one strand is amplified in greater amount than the other.

The skill of the invention lies in the analysis of the bisulfite treated DNA. In other forms of methylation analysis a purification step is required before further analysis of the methylation patterns can occur. However, one of the advantages of the invention is that the bisulfite treated DNA amplification products may be left in solution.

The present invention includes a method to distinguish a methylated sequence from an unmethylated sequence. In a preferred embodiment this includes the analysis of methylation patterns at CpG sites, and any regulatory regions within the genome. Subsequent to the PCR amplification of the bisulfite converted sequence, oligonucleotides are contacted with the bisulfite treated DNA. Such contact may take any form. The oligonucleotides consist of multiple pairs. Each of said pairs of oligonucleotides annealing to a specific CpG site that is to be analysed within the target sequence. Each species of oligonucleotide is covalently labelled using a unique fluorescent tag. Each member of each pair is specific for targeting a particular methylation specific bisulfite treated conformation of the target site. In a preferred embodiment the concentration of said labelled oligonucleotides may be calculated such that the concentration of each species is not in excess of the concentration of the target DNA. The design of oligonucleotides specific to bisulfite treated DNA sequence is within the state of the art.

The oligonucleotides and amplificate may then be brought together under conditions conducive to hybridisation. This is within the skill of the art. Hybridisation conditions are selected so as to limit hybridisation of only a single oligonucleotide species to each target CpG site . In the preferred embodiment one species of said oligonucleotide hybridises to the target sequence. The fluorescence polarisation is then measured for each fluorescent label.

In a further preferred embodiment said oligonucleotide probes may be utilised as extension primers for the enzymatic amplification of the nucleic acid. After hybridisation of the oligonucleotide to the target sequence the oligonucleotide may be extended by the addition of a DNA polymerase and nucleotides. In this embodiment the increase in mass of the hybridised nucleotide further increases the sensitivity of the assay.

In a further preferred embodiment the fluorescence polarisation of the labelled oligonucleotides may be measured prior to hybridisation with the amplificate and again after hybridisation with the amplificate. Hybridisation of the oligonucleotide may then be observed by an increase in fluorescence polarisation after hybridisation. This method allows the analysis of nucleic acids that may not be amenable to standardisation of conditions.

Also disclosed is a diagnostic kit. The components of said kit should comprise receptacles for the following in sufficient quantities to carry out the examples:
1) Reagents for the bisulfite conversion of sample DNA to bisulfite sequence;
2) Fluorophore labelled nucleic acid oligonucleotides;
3) Instructions for use.

The term 'instructions for use' should cover a tangible expression describing the reagent concentrations for the assay method, parameters such as the relative amounts of reagents to be combined, maintenance times for re-agents/sample mixtures, temperature, buffer conditions and such like.

A wide variety of fluorophores are suitable for use in fluorescence polarisation techniques. The selection of appropriate fluorophores is within the skill of the art. Preferred fluorophores include, but are not limited to, 5'carboxyfluorescein (FAM); 6-carboxy-X-rhodamine (ROX); N,N,N',N',-tetramethyl-6-carboxy-X-rhodamine (TMR); and BODIPY-Texas Red (BTR); CY5, CY3, FITC, DAPI, HEX, and TET. The design of suitable oligonucleotides, and their synthesis with fluorescent labels is within the skill of the art. In a preferred embodiment of the invention, the length of the linkers used to attach the fluorophores to the bases of the oligonucleotides are kept to a minimum, while achieving maximum rigidity. Short and/or rigid linkers keep the movement of the fluorophore relative to the oligonucleotide to a minimum. This allows an increase in the sensitivity of the assay.

The sensitivity of the assay may also be increased limiting the rotational motility of the fluorophore by increasing the mass of the Type B oligonucleotides or the DNA amplificate. The attachment of mass labels is within the skill of the art and has been described in detail in Patent Application WO0023785, which is hereby incorporated for reference. The present invention covers two methods of achieving this. In a first embodiment, the bisulfite treated DNA amplificate is immobilised by attachment to a surface or macromolecule, prior to hybridisation with the fluorescently labelled oligonucleotides.
The DNA may be applied by any chemical or physical means known in the state of the art.

In a further preferred embodiment the method may be performed in an unhomogenous manner, i.e. involving a separation step. After hybridisation of the oligonucleotide probe to the amplificate and measurement of fluorescence polarisation, the probe may be removed, for example by heat denaturing the DNA duplex followed by washing. The surface bound DNA may then be hybridised to another oligonucleotide probe. Said procedure may be repeated a multiplicity of times. This method allows one the analysis of one DNA fragment by multiple sets of probes.

In a preferred embodiment the Type B oligonucleotides may be immobilised to a surface or solid phase prior to hybridisation.

In a further preferred embodiment the method may be performed in an unhomogenous manner, i.e. involving a separation step. After hybridisation of the amplificate to the oligonucleotide probe and measurement of fluorescence polarisation, the amplificate may be removed, for example by heat denaturing the DNA duplex followed by washing.

The surface bound probes may then be hybridised to another DNA fragment. Said procedure may be repeated a multiplicity of times. This method allows one set of oligonucleotide probes to be used for the analysis of multiple sets of DNA fragments.

Measurement of fluorescence polarisation may be carried out using commercially available fluorimeters. It is to be understood that fluorescence polarisation includes all methods comprising the analysis of plane polarised light emitted from the fluorophore when excited by plane polarised light.

It is anticipated that the method will be used for the high throughput analysis of genomic DNA samples. Therefore the claims also cover a method for the analysis of data using a computing device. In a preferred embodiment said device may include one or more databases. In a further preferred embodiment said device may include one or more learning algorithms.

### Description of Diagrams

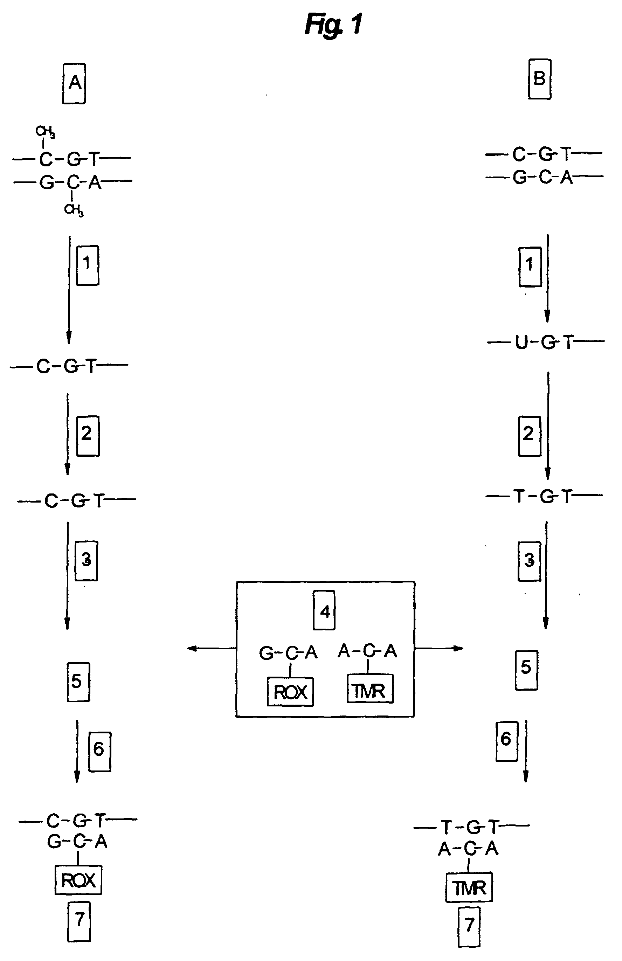

### Figure 1: Hybridisation Assay

A - Genomic DNA fragment, target sequence methylated.
B - Genomic DNA fragment, target sequence unmethylated.

The genomic DNA is modified such that unmethylated cytosine bases are converted into uracil (1). The target site is amplified by polymerase chain reaction (2). The amplification may be such that only one strand is amplified. Amplified sequence differs from genomic sequence in that methylated cytosine replaced with thymine, therefore double strands of DNA sequence may no longer be complementary.

The amplicon is then contacted with the fluorescently labelled oligonucleotide pairs (3), in this case ROX and TMR. The fluorescence polarisation of the labels is then measured (4). The complementary oligonucleotide is then hybridised to the target site (5). The fluorescence polarisation is then measured again (6).

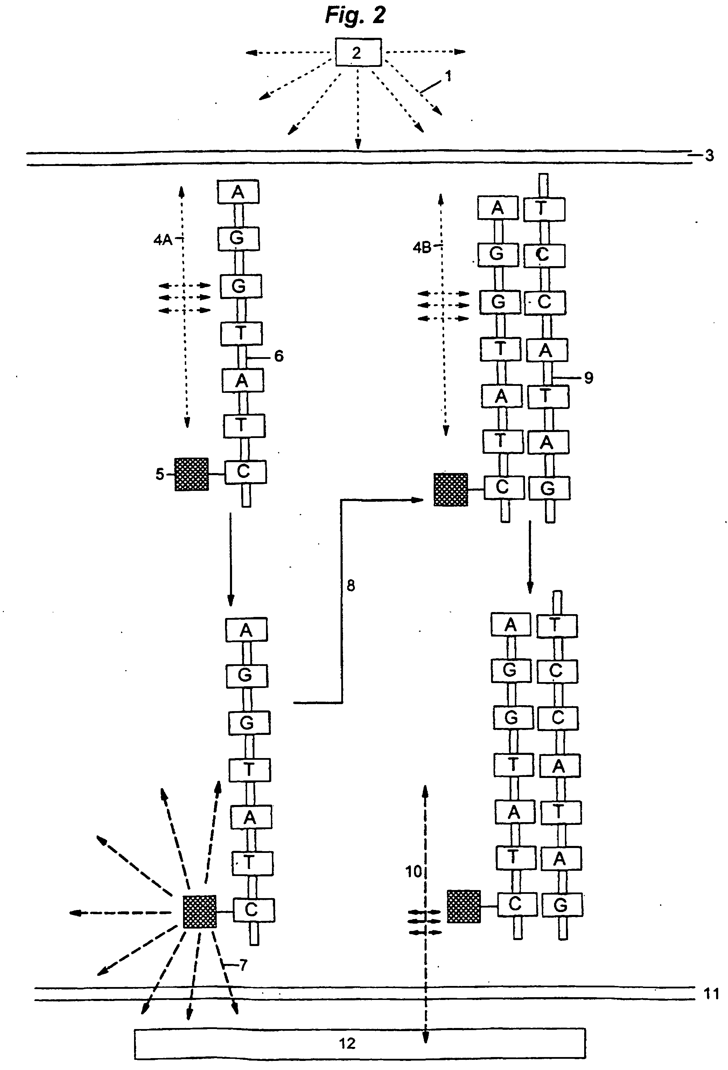

Figure 2: Measurement of fluorescence polarisation Unpolarised light (1) from a light source (2) is passed though polarisation and colour filters (3). The plane polarised light (4A) is then passed through the reaction solution prior to oligonucleotide hybridisation. The polarised light excites the fluorescent label (5) attached to the oligonucleotide (6) such that the fluorescent label emits light (7). As the oligonucleotide is free in solution, the fluorescent label has a high degree of motion and emissions are not polarised (7) . The fluorescence polarisation of (7) may then be measured. In such an embodiment the emissions are passed through polarisation and colour filters (10). The emissions are measured using a fluorimeter (11).

Hybridisation conditions are applied (8). The labelled oligonucleotide is hybridised to a larger nucleic acid (9). Due to the increase in molecular weight the fluorescent label has a lower degree of motion. Therefore, when excited by the plane polarised light (4B), the emissions (10) have a higher degree of polarisation. The emissions are then passed through polarisation and colour filters (11). The emissions are measured using a fluorimeter (12).

## Claims

1. A method for the analysis of the methylation of specific cytosine bases in genomic DNA samples, **characterised by** the fact that the following steps are implemented:
(a) the genomic DNA is chemically treated in such a manner that cytosine is converted into uracil or a similar acting base regarding the base pairing behaviour in the DNA duplex, 5 methylcytosine however remains basically unmodified;
(b) the chemically treated DNA is amplified using at least one oligonucleotide (type A) as primer in a polymerase reaction, whereby the two strands of the polymerase reaction product are manufactured in unequal quantities;
(c) the amplificate is hybridised with one or more pairs of oligonucleotides (type B), which hybridise to the positions which are to be examined regarding their methylation status in the genomic DNA sample whereby one oligonucleotide of each pair hybridises preferentially in each case if in the genomic DNA sample the position was methylated, while the other oligonucleotide of the pair hybridises preferentially, if the position was unmethylated. Each oligonucleotide of a pair is labelled with a unique fluorescent label;
(d) the fluorescence polarisation characteristics of the solution are measured, whereby for each fluorescent label used one determines the degree of polarisation.

2. A method according to claim 1 wherein the hybridised oligonucleotide is extended by means of deoxynucleotides and a polymerase.

3. A method according to claims 1 and 2 whereby the fluorescence polarisation of the fluorophore labelled oligonucleotides is measured before hybridisation with the amplificate and again after hybridisation with the amplificate.

4. A method according to claims 3 whereby the oligonucleotide hybridisation is detected by an increase in fluorescence polarisation.

5. A method according to claim 1 wherein said fluorophore is selected from the group consisting of 5'carboxyfluorescein, 6-carboxy-X-rhodamine, N,N,N',N',-tetramethyl-6-carboxy-X-rhodamine, BODIPY,Texas Red, Cy3, Cy5, FAM, FITC, DAPI, HEX, and TET.

6. A method according to claims 1 whereby the DNA sample is cleaved prior to bisulfite treatment with restriction endonucleases.

7. A method according to claim 1 whereby the enzymatic amplification of the chemically treated DNA is such that only one strand of the DNA sample is amplified.

8. A method according to claim 1 whereby the DNA sample has been isolated from mammalian sources

9. A method according to claim 1 wherein the fluorescence polarisation of the enzymatically amplified DNA is measured directly from the container in which the amplification reaction was carried out.

10. A method according to claim 1 the Type B oligonucleotide is immobilised on a surface prior to hybridisation with the amplificate.

11. A method according to claim 1 wherein the bisulfite treated DNA is bound to a surface prior to hybridisation with the Type B oligonucleotide.

12. A method according to claims 11 and 12 whereby the surface comprises silicon, glass, polystyrene, aluminium, steel, iron, copper, nickel, silver or gold.

13. A method according to claims 1 and 2 whereby the information generated about the methylation status at the target site is provided to a computing device comprising one or more databases.

14. A method according to claims 1 and 2 whereby the information generated about the methylation status at the target site is provided to a computing device comprising one or more learning algorithms.

## Patentansprüche

1. Verfahren zur Analyse der Methylierung spezifischer Cytosin-Basen in genomischen DNA-Proben, **dadurch gekennzeichnet, dass** die folgenden Schritte ausgeführt werden:
(a) die genomische DNA wird chemisch derart behandelt, dass Cytosin zu Uracil oder einer bezüglich des Basenpaarungsverhaltens in der DNA-Duplex ähnlich reagierenden Base umgesetzt wird, 5-Methylcytosin jedoch im Wesentlichen unmodifiziert bleibt;
(b) die chemisch behandelte DNA wird amplifiziert, wobei mindestens ein Oligonukleotid (Typ A) als Primer in einer Polymerase-Reaktion verwendet wird und die beiden Stränge der Polymerase-Reaktionsprodukte in ungleichen Mengen hergestellt werden;
(c) das Amplifizierungsprodukt wird mit einem oder mehreren Paaren von Oligonukleotiden (Typ B) hybridisiert, welche an die Positionen hybridisieren, welche bezüglich ihres Methylierungs-Status in der genomischen DNA-Probe untersuchen werden sollen, wobei ein Oligonukleotid eines jeden Paares in jedem Fall-bevorzugt hybridisiert, wenn in der genomischen DNA-Probe die Position methyliert war, während das andere Oligonukleotid des Paares bevorzugt hybridisiert, wenn die Position unmethyliert war. Jedes Oligonukleotid eines Paares ist mit einem einzelnen Fluoreszenzmarker markiert;
d) die Fluoreszenzpolarisations-Eigenschaften der Lösung werden gemessen, wobei man für jeden verwendeten Fluoreszenzmarker den Polarisationsgrad bestimmt.

2. Verfahren nach Anspruch 1, worin das hybridisierte Oligonukleotid mittels Deoxynukleotiden und einer Polymerase verlängert wird.

3. Verfahren nach einem der Ansprüche 1 und 2, wobei die Fluoreszenzpolarisation der fluorophormarkierten Oligonukleotide vor der Hybridisierung mit dem Amplifizierungsprodukt und nochmals nach der Hybridisierung mit dem Amplifizierugsprodukt gemessen wird.

4. Verfahren nach Anspruch 3, wobei die Oligonukleotid-Hybridisierung durch einen Anstieg der Fluoreszenzpolarisation detektiert wird.

5. Verfahren nach Anspruch 1, worin das Fluorophor ausgewählt wird aus der Gruppe umfassend 5'-Carboxyfluorescein, 6-Carboxy-X-rhodamin, N,N,N',N'-Tetramethyl-6-carboxy-X-rhodamin, BODIPY, TexasRed, Cy3, Cy5, FAM, FITC, DAPI, HEX und TET.

6. Verfahren nach Anspruch 1, wobei die DNA-Probe vor der Bisulfit-Behandlung mit Restriktionsendonukleasen gespalten wird.

7. Verfahren nach Anspruch 1, wobei die enzymatische Amplifizierung der chemisch behandelten DNA so durchgeführt wird, dass nur ein Strang der DNA-Probe amplifiziert wird.

8. Verfahren nach Anspruch 1, wobei die DNA-Probe aus Säugetier-Quellen.

9. Verfahren nach Anspruch 1, worin die Fluoreszenzpolarisation der enzymatisch amplifizierten DNA direkt in dem Behälter gemessen wird, in dem die Amplifizierungs-Reaktion durchgeführt wurde.

10. Verfahren nach Anspruch 1, worin das Typ-B-Oligonukleotid vor der Hybridisierung mit dem Amplifizierungsprodukt an einer Oberfläche immobilisiert wird.

11. Verfahren nach Anspruch 1, worin die mit Bisulfit behandelte DNA vor der Hybridisierung mit dem Typ-B-Oligonukleotid an eine Oberfläche gebunden wird.

12. Verfahren nach einem der Ansprüche 11 und 12, worin die Oberfläche Silikon, Glas, Polystyrol, Aluminium, Stahl, Eisen, Kupfer, Nickel, Silber oder Gold umfasst.

13. Verfahren nach einem der Ansprüche 1 und 2, worin die über den Methylierungs-Status der Target-Stelle erzeugte Information einer Rechneranlage zugeführt wird, welche eine oder mehrere Datenbanken umfasst.

14. Verfahren nach einem der Ansprüche 1 und 2, wobei die über den Methylierungs-Status der Target-Stelle erzeugte Information einer Rechneranlage zugeführt wird, welche einen oder mehrere lernende Algorithmen umfasst.

## Revendications

1. Procédé pour l'analyse de la méthylation de bases de cytosine spécifiques dans des échantillons d'ADN génomique, **caractérisé par le fait que** les étapes suivantes sont mises en oeuvre :
(a) l'ADN génomique est traité chimiquement d'une manière telle que la cytosine est convertie en uracile ou une base à action similaire concernant le comportement d'appariement de base dans le duplex d'ADN, la 5-méthylcytosine reste cependant principalement non modifiée ;
(b) l'ADN traité chimiquement est amplifié en utilisant au moins un oligonucléotide (type A) comme amorce dans une réaction dé polymérase, moyennant quoi les deux brins du produit de réaction de polymérase sont fabriqués en quantités inégales ;
(c) le produit d'amplification est hybridé avec une ou plusieurs paires d'oligonucléotides (type B), qui s'hybrident aux positions qui doivent être examinées concernant leur état de méthylation dans l'échantillon d'ADN génomique moyennant quoi un oligonucléotide de chaque paire s'hybride de préférence dans chaque cas si dans l'échantillon d'ADN génomique la position était méthylée, alors que l'autre oligonucléotide de la paire s'hybride préférentiellement si la position était non méthylée. Chaque oligonucléotide d'une paire est marqué avec un marqueur fluorescent unique ;
(d) les caractéristiques de polarisation de fluorescence de la solution sont mesurées, moyennant quoi pour chaque marqueur fluorescent utilisé on détermine le degré de'polarisation.

2. Procédé selon la revendication 1, dans lequel l'oligonucléotide hybridé est prolongé au moyen de désoxynucléotides et d'une polymérase.

3. Procédé selon les revendications 1 et 2 moyennant quoi la polarisation de fluorescence des oligonucléotides marqués au fluorophore est mesurée avant l'hybridation avec le produit d'amplification et encore après l'hybridation avec le produit d'amplification.

4. Procédé selon la revendication 3, moyennant quoi l'hybridation d'oligonucléotide est détectée par un accroissement de polarisation de fluorescence.

5. Procédé selon la revendication 1, dans lequel ledit fluorophore est choisi dans le groupe constitué de la 5'-carboxyfluorescéine, la 6-carboxy-X-rhodamine, la N,N,N',N'-tétraméthyl-6-carboxy-X-rhodamine, BODIPY, rouge Texas, Cy3, Cy5, FAM, FITC, DAPI, HEX, et TET.

6. Procédé selon la revendication 1, moyennant quoi l'échantillon d'ADN est clivé avant le traitement au bisulfite avec des endonucléases de restriction.

7. Procédé selon la revendication 1, moyennant quoi l'amplification enzymatique de l'ADN traité chimiquement est telle que seul un brin de l'échantillon d'ADN est amplifié.

8. Procédé selon la revendication 1, moyennant quoi l'échantillon d'ADN a été isolé à partir de sources mammifères.

9. Procédé selon la revendication 1, dans lequel la polarisation de fluorescence de l'ADN amplifié par voie enzymatique est mesurée directement à partir du conteneur dans lequel la réaction d'amplification a été effectuée.

10. Procédé selon la revendication 1, l'oligonucléotide de type B est immobilisé sur une surface avant l'hybridation avec le produit d'amplification.

11. Procédé selon la revendication 1, dans lequel l'ADN traité au bisulfite est lié à une surface avant l'hybridation avec l'oligonucléotide de type B.

12. Procédé selon les revendication 11 et 12, moyennant quoi la surface comprend du silicium, du verre, du polystyrène, de l'aluminium, de l'acier, du fer, du cuivre, du nickel, de l'argent ou de l'or.

13. Procédé selon les revendications 1 et 2, moyennant quoi l'information produite sur l'état de méthylation au site cible est fourni à un dispositif informatique comprenant une ou plusieurs bases de données.

14. Procédé selon les revendications 1 et 2, moyennant quoi l'information produite sur l'état de méthylation au site cible est fourni à un dispositif informatique comprenant un ou plusieurs algorithmes d'apprentissage.
